# EUROPÄISCHE PATENTSCHRIFT

(11) **EP 0 687 162 B1**
(45) Veröffentlichungstag und Bekanntmachung des Hinweises auf die Patenterteilung: **14.01.1998**
(21) Anmeldenummer: 94909104.5
(22) Anmeldetag: 02.03.1994
(51) Int. Cl.: A61B 5/103, A61B 5/107, G01B 21/30

(54) **VORRICHTUNG UND VERFAHREN ZUR MESSUNG EINER DREIDIMENSIONALEN OBERFLÄCHENSTRUKTUR**
DEVICE AND METHOD FOR MEASURING THE DIMENSIONS OF A THREE-DIMENSIONAL SURFACE STRUCTURE
PROCEDE ET DISPOSITIF PERMETTANT DE MESURER UNE STRUCTURE SUPERFICIELLE TRIDIMENSIONNELLE

(30) Priorität: 03.03.1993 DE 9303102 U
(43) Veröffentlichungstag der Anmeldung: 20.12.1995
(73) Patentinhaber: COURAGE + KHAZAKA ELECTRONIC GmbH, D-50829 Köln (DE)
(72) Erfinder: KHAZAKA, Gabriel, D-50859 Köln (DE); COURAGE, Wilfried, D-50937 Köln (DE)
(74) Vertreter: Dallmeyer, Georg, Dipl.-Ing.
(86) Internationale Anmeldenummer: EP9400612
(87) Internationale Veröffentlichungsnummer: WO9420019

(56) Entgegenhaltungen:
- FR-A- 2 658 713
- PATENT ABSTRACTS OF JAPAN vol. 8, no. 144 (P-284) (1581) 5. July 1984 & JP,A,59 043 358 (KANEBO KK) 10 March 1984

## Beschreibung

Die Erfindung betrifft eine Vorrichtung zur Messung einer dreidimensionalen Oberflächenstruktur nach dem Oberbegriff des Anspruchs 1 sowie ein Verfahren nach dem Oberbegriff des Anspruchs 9.

Ein Einsatzgebiet derartiger Meßgeräte ist die Untersuchung der Hautoberfläche für die Hautkosmetik. In diesem Bereich ist es erwünscht, die Hautstruktur möglichst exakt dreidimensional zu erfassen, wobei zwecks Nachweis der Wirkung von Hautbehandlungsmitteln es entscheidend auf eine sehr hohe Auflösung der Meßwerte ankommt, um Änderungen der Hautstruktur im zeitlichen Abstand feststellen zu können.

Andere Anwendungsbeispiele aus der Technik betreffen Holzoberflächen oder Metalloberflächen.

Es ist bereits bekannt, von der Hautoberfläche Silikonabdrücke anzufertigen und anschließend den Abdruck schräg zu belichten, um entsprechend dem Schattenfall eine Information über die dreidimensionale Struktur zu erhalten. Eine solche Messung hat den Nachteil, von Entfernung und Lichteinfallwinkel der Lichtquelle abhängig zu sein, wobei des weiteren die Auswertung sehr zeitaufwendig ist.

Aus der FR-A-2658713 ist ein Verfahren zur Analyse der Hautoberfläche bekannt, bei dem als Abdruckmaterial die Verwendung von Silikon vorgeschlagen wird.

Es besteht auch die Möglichkeit, den Silikonabdruck mit Laser abzutasten, was jedoch kostenaufwendig ist.

Eine weitere Möglichkeit besteht darin, den Abdruck mit einer Diamantspitze abzutasten, um ein dreidimensionales Bild der Hautoberfläche zu erzeugen, wobei die Genauigkeit der dreidimensionalen Abbildung von der Rasterauflösung der Abtastung abhängig ist. Eine derartige Messung ist ebenfalls sehr zeitaufwendig und durch die Abhängigkeit von dem Raster der Abtastung nicht ausreichend genau. Ein derartiges Verfahren ist beispielsweise aus der DE 27 19 341 C bekannt.

Aus dem Dokument JP-A 59043358 ist eine Vorrichtung zur Messung einer Hautoberfläche bekannt, die eine transparente Kunststoffträgerplatte mit einer Klebeschicht aufweist, sowie eine auf dieser Klebeschicht aufgeklebte transparente Kunststoffolie. Diese Kunststoffolie ist zur Bildung eines Meßfeldes kreisförmig ausgespart, wobei das Meßfeld vor Gebrauch mit einer Schutzfolie abgedeckt ist. Für die Probenahme wird ein anfangs flüssiges Abdruckmaterial auf die Hautoberfläche geträufelt und nach dem Aushärten durch Verkleben mit dem Meßfeld von der Hautoberfläche abgenommen. Das mit dem Meßfeld verklebte Abdruckmaterial kann dann in einem Mikroskop untersucht werden.

Zusammenfassend besteht der Nachteil der bekannten Meßvorrichtung darin, daß sich die Messungen nur näherungsweise durchführen lassen, daß die Reproduzierbarkeit der Meßwerte gering ist, und daß der Zeitbedarf zu hoch ist.

Der Erfindung liegt die Aufgabe zugrunde, eine Vorrichtung zur Messung einer dreidimensionalen Oberflächenstruktur zu schaffen, die mit geringem Zeitaufwand dreidimensional auswertbare, in hohem Maße reproduzierbare und kalibrierbare Meßwerte liefert.

Zur Lösung dieser Aufgabe dienen die Merkmale des Anspruchs 1 bzw. 9.

Die Erfindung sieht vor, daß die Trägerplatte ein Meßfeld aufweist, das in vorbestimmten Abstand von der Meßoberfläche gehalten ist und daß das in das Meßfeld einzubringende Silikonmaterial gleichmäßig eingefärbt ist, wobei die Trägerplatte mindestens einen von dem Meßfeld wegführenden Abflußkanal für überschüssiges Silikonmaterial aufweist. Auf diese Weise ist gewährleistet, daß die Meßoberfläche nicht beim Herstellen des Silikonabdrucks verformt wird, wodurch sich Meßfehler ergeben würden. Vielmehr ist durch den von der Meßfläche wegführenden Abflußkanal sichergestellt, daß der Silikonabdruck weitestgehend druckfrei erzeugt werden kann. Das Meßfeld wird in vorbestimmten Abstand von der Meßoberfläche gehalten. Dadurch ist die Schichtstärke des Silikonabdrucks auf eine Dicke eingestellt, die es ermöglicht, die gesamte Tiefe der dreidimensionalen Oberflächenstruktur zu erfassen, wobei gleichzeitig sichergestellt ist, daß die Schichtstärke nicht zu dick wird, was die Auswertung aufgrund einer Lichtintensitäts- bzw. Lichtabsorptionsmessung erschweren würde. Da das Silikonmaterial gleichmäßig eingefärbt ist, ist die Lichtabsorption der Silikonmasse ein direktes Maß der Schichtstärke des Silikonabdrucks. Mit einer geeigneten optischen Auswerteeinrichtung ist es mit einem solchen Silikonabdruck möglich, schnell auswertbare, in hohem Maße reproduzierbare und kalibrierbare dreidimensionale Meßwerte zu erzielen.

Bei einem Ausführungsbeispiel ist vorgesehen, daß die Trägerplatte aus einer dünnen transparenten Scheibe und einer doppelseitig klebenden Folie besteht, die als Abstandhalter dient und in der das Meßfeld und mindestens ein Abschlußkanal ausgestanzt sind. Eine derartige aus zwei Teilen zusammengesetzte Trägerplatte läßt sich leicht auf Hautoberflächen applizieren und ist besser an Hautoberflächen adaptierbar. Insbesondere läßt sich eine derartige Trägerplatte mit verhältnismäßig kleiner Dimension herstellen, wodurch die Beeinflussung der Meßoberfläche durch die Trägerplatte reduziert wird.

Bei einem zweiten Ausführungsbeispiel ist vorgesehen, daß innerhalb des Meßfelds eine zu der Meßoberfläche gerichtete stufenförmige Kalibriereinrichtung mit mindestens zwei definierten Stufenabständen vorgesehen ist. Die von der Oberfläche des Meßfelds ausgehenden Abstufungen der Kalibriereinrichtung ermöglichen es in vorteilhafter Weise, bestimmte Helligkeitswerte des Silikonmaterials entsprechenden Tiefenwerten exakt zuzuordnen, so daß in vorteilhafter Weise eine Kalibrierung der Meßdaten möglich ist und dreidimensionale Tiefenwerte mit hoher Genauigkeit und Reproduzierbarkeit meßbar sind.

Die Kalibrierung ermöglicht es in vorteilhafter Weise eine mögliche Nicht-Linearität der Meßsignale auszugleichen. Der Silikonabdruck auf der Trägerplatte wird vorzugsweise mit parallelem Licht angestrahlt, wobei das gleichmäßig durchgefärbte Silikonmaterial je nach Schichtstärke einen unterschiedlichen Helligkeitswert für einen Detektor, z.B. eine Optik mit CCD-Einrichtung, erzeugt.

Vorzugsweise ist vorgesehen, daß das Silikonmaterial mit einer solchen gleichmäßigen Intensität eingefärbt ist, daß für jeden Stufensprung der Kalibriereinrichtung ein mit hoher Auflösung meßbarer Helligkeitsunterschied entsteht. Dabei ist die Absorptionsdichte des Silikonmaterials auf eine maximale Schichtstärke von ca. 1 mm abgestimmt.

Auf diese Weise lassen sich mit Hilfe von Detektoren bezogen auf eine Schichtstärke von ca. 1 mm 256 Helligkeitswerte unterscheiden, so daß die Tiefenbestimmung mit äußerst hoher Genauigkeit und Reproduzierbarkeit erfolgen kann.

Vorzugsweise ist die Trägerplatte in einer Halterung angeordnet, die in Diaprojektoren einsetzbar ist. Die Einfallszonen der Trägerplatte in einem Diarahmenformat ermöglicht die schnelle Auswertung der Abdrücke mit Hilfe von Videoauswerteeinrichtungen.

Neben dem Meßfeld der Trägerplatte können Abflußkanäle allseitig zur Rückseite der Trägerplatte hindurchgeführt sein. Die Abflußkanäle ermöglichen den Abtransport von überschüssigem Silikonmaterial und verhindern dadurch einen Druckaufbau im Bereich der Meßoberfläche. Des weiteren wird der Silikonabdruck auf der Trägerplatte vorteilhaft verankert.

Im folgenden wird unter Bezugnahme auf die Zeichnungen ein Ausführungsbeispiel der Erfindungen näher erläutert.

Es zeigen:
- Fig. 1: Folien für eine Trägerplatte eines ersten Ausführungsbeispiels,
- Fig. 2: ein Schnitt entlang der Linie II-II in Fig. 1,
- Fig. 3: die Applikation des ersten Ausführungsbeispiels auf der Meßoberfläche,
- Fig. 4: die Herstellung des Abdrucks auf der Meßoberfläche,
- Fig. 5: ein Schnitt entlang der Linie entlang der Linie V-V in Fig. 4,
- Fig. 6: das Aufbringen der Trägerplatte gemäß dem ersten Ausführungsbeispiel auf einen Rahmen,
- Fig. 7: ein Schnitt entlang der Linie VII-VII in Fig. 6,
- Fig. 8: eine Draufsicht auf die von der Meßoberfläche abgewandte Seite eines zweiten Ausführungsbeispiels einer in einem Rahmen eingefaßten Trägerplatte,
- Fig. 9: die der Meßoberfläche zugewandte Seite des Ausführungsbeispiels gemäß Fig. 8 und
- Fig. 10: einen Schnitt entlang der Linie X-X in den Fig. 8 und 9.

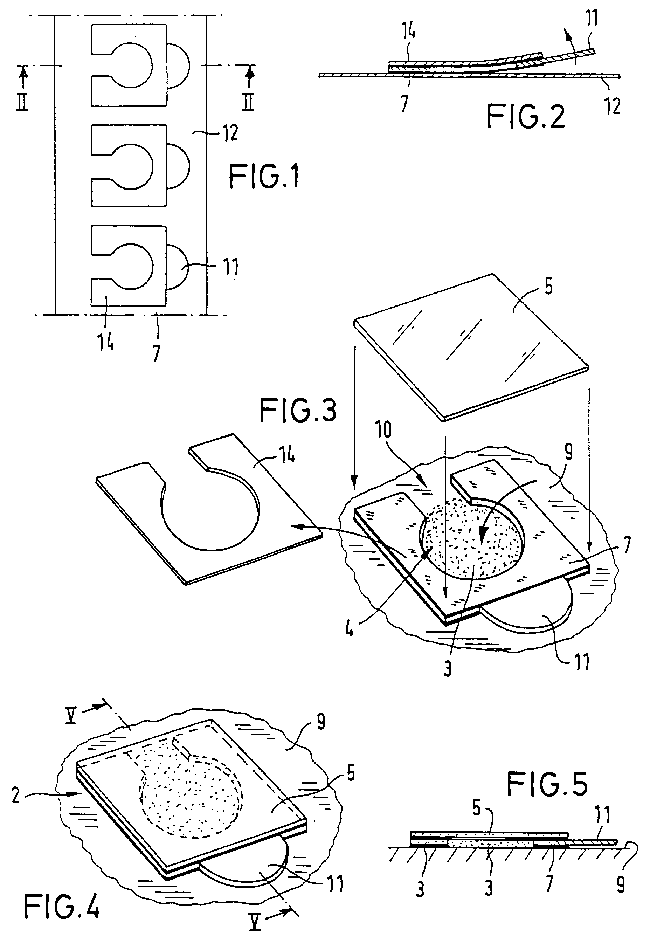

Fig. 3 zeigt die Applikation eines ersten Ausführungsbeispiels auf einer Meßoberfläche, z.B. einer Hautoberfläche 9. Hierzu werden zunächst, wie am besten aus Fig. 1 ersichtlich, auf einem Folienträger 12 befindliche doppelseitig klebende Folien 7 mit Hilfe einer Lasche 11 von dem Folienträger 12 entfernt und an der gewünschten Meßstelle auf die Hautoberfläche 9 geklebt.

Die Folie 7 besteht aus einer transparenten flexiblen Kunststoffolie mit einer Dicke von ca. 0,4 bis 0,7 mm, vorzugsweise ca. 0,5 mm, die in ihrer Mitte eine im wesentlichen kreisförmige Ausstanzung aufweist, die ein im wesentlichen kreisförmiges Meßfeld 4 begrenzt. Eine weitere Ausstanzung schafft einen Abflußkanal 10 von dem Meßfeld 4 nach außen.

Die auf dem Folienträger 12 angeordneten Folien 7 weisen ein Abdeckpapier 14 auf, das nach dem Aufbringen der Folie 7 auf die Meßoberfläche, wie in Fig. 3 dargestellt, entfernt werden kann.

Nach dem Entfernen des Abdeckpapiers 14 werden mehrere Tropfen flüssiges Silikonmaterial 3 auf das von der Folie 7 umgrenzte Meßfeld 4 auf die Hauptoberfläche 9 gegeben. Anschließend wird eine transparente Scheibe 5, die gemeinsam mit der Folie 7 die Trägerplatte 2 des ersten Ausführungsbeispiels bildet auf die Folie 7 aufgesetzt, die aufgrund der Klebeschicht auf der Folie 7 auf dieser fest anhaftet.

Die Scheibe 5 kann aus einem dünnen vorzugsweise entspiegeltem Glas bestehen. Beim Aufsetzen der Scheibe 5 wird überschüssiges Silikonmaterial über den Abflußkanal 10 nach außen geführt, wodurch ein druckfreier und daher unverfälschter Silikonabdruck von der Meßoberfläche herstellbar ist. Bei einer Scheibe 5 aus Glas genügt das Eigengewicht der Scheibe, um das überschüssige Silikonmaterial über den Abflußkanal 10 herauszudrücken. Nach dem Aushärten des Silikonmaterials 3 kann mit Hilfe der Lasche 11 die Trägerplatte 2, d.h. die Folie 7 gemeinsam mit der Scheibe 5 und dem Silikonabdruck von der Hautoberfläche 9 abgezogen werden und in eine optische Auswerteeinrichtung gegeben werden.

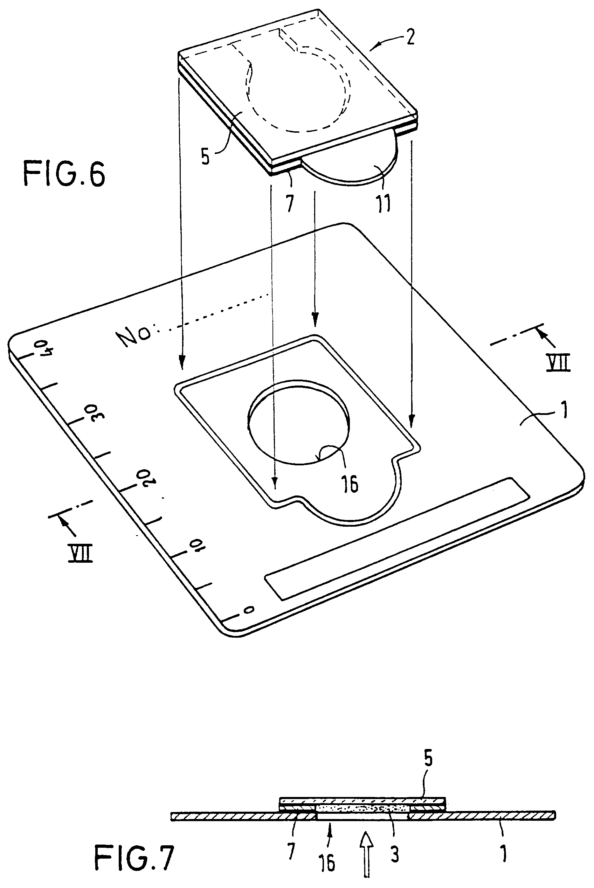

Die der Hautoberfläche zugewandte Klebefläche der Trägerplatte 2 kann dazu verwendet werden, die Trägerplatte 2 auf einen Rahmen 1 aufzukleben, der beispielsweise in Diaprojektoren oder Videoauswerteeinrichtungen eingesetzt werden kann und der in geeigneter Weise beschriftet werden kann. Hierzu weist der Rahmen, wie aus Fig. 6 ersichtlich, in seiner Mitte eine kreisrunde Öffnung 16 auf, die dem Meßfeld 4 angepaßt ist und ein Durchleuchten der Trägerplatte 2 mit dem Silikonabdruck ermöglicht.

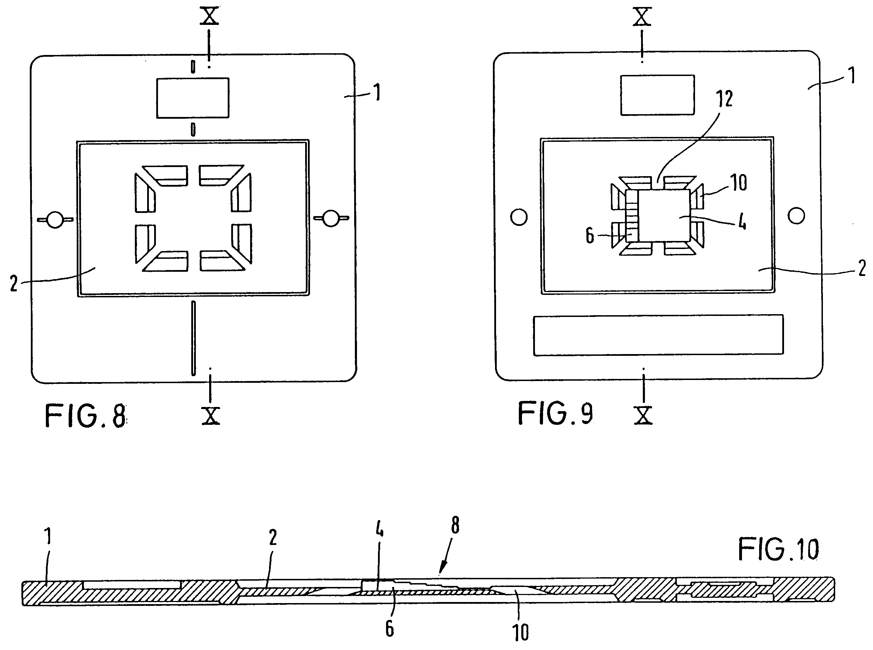

Fign. 8, 9 und 10 zeigen eine in einem Rahmen 1 eingefaßte Trägerplatte 2 eines zweiten Ausführungsbeispiels mit integrierter Kalibriereinrichtung. Die anfangs noch flüssige Silikonschicht ermöglicht, einen Silikonabdruck einer dreidimensionalen Oberflächenstruktur, insbesondere von Hautfalten einer menschlichen Hautoberfläche, zu erzeugen.

Der Rahmen 1 entspricht hinsichtlich seiner Abmessungen exakt handelsüblichen Kleinbild-Diarähmchen, so daß der Silikonabdruck auf der Trägerplatte 2 mit Hilfe von Diaprojektoren und Videoauswerteeinrichtungen ausgewertet werden kann.

Die Trägerplatte 2 weist auf der der Meßoberfläche zugewandten Seite in der Mitte das Meßfeld 4 auf, von dem an einer Seite des Meßfeldes 4 eine Kalibriereinrichtung 6 in Richtung auf die Meßoberfläche absteht.

Die Kalibriereinrichtung weist mehrere Abstufungen 8 mit exakt definierten Stufenabständen auf, so daß im Bereich der Kalibriereinrichtung 6 das gleichmäßig durchgefärbte Silikonmaterial stufenförmig in unterschiedlicher Schichtstärke vorliegt. Beim Durchleuchten des Silikonabdrucks können mit Hilfe der Abstufungen bestimmte Helligkeitswerte des Abdrucks exakten Tiefenmeßwerten zugeordnet werden. Die in dem Meßfeld vorzugsweise digital ausgewerteten Helligkeitsunterschiede können mit Hilfe der Kalibriereinrichtung 6 exakt kalibriert werden, so daß eine in hohem Maße reproduzierbare und genaue Tiefenbestimmung der Oberflächenstruktur möglich ist.

Für das erste Ausführungsbeispiel gemäß den Fign. 1 bis 7 ist es auch möglich, eine separate Kalibriereinrichtung 6 zu verwenden, da es nicht erforderlich ist, für jeden Abdruck auch eine Kalibrierskala herzustellen. So reicht es beispielsweise aus, für jede eingefärbte Silikoncharge einen Kalibrierabdruck zu erzeugen.

Mit Hilfe einer CCD-Detektor-Einrichtung ist die Unterscheidung von 256 Helligkeitswerten möglich, wobei die Tiefenwerte der Oberflächenstruktur üblicherweise eine Strukturdifferenz von maximal ca. 0,5 mm aufweisen.

Die gleichmäßige Einfärbung des Silikonmaterials ist auf die maximale Schichtstärke der Silikonschicht von ca. 1 mm abgestimmt, so daß die Auflösung der Helligkeitswerte für den interessierenden Meßbereich optimiert ist.

Die der Meßoberfläche abgewandte Seite der Trägerplatte 2 ist vorzugsweise zumindest im Bereich des Meßfeldes 4 entspiegelt. Die Trägerplatte 2 ist ungefärbt und besteht aus Glas oder Kunststoff. Sie kann entspiegelt sein.

Gemäß dem zweiten Ausführungsbeispiel verlaufen vom äußeren Rand des Meßfeldes 4 radial nach außen sich erstreckende Abflußkanäle 10 schräg durch die Trägerplatte 2 hindurch. Die schlitzförmigen Abflußkanäle 10 erweitern sich dabei radial nach außen. Die Abflußkanäle 10 ermöglichen den Abfluß von überflüssigem Silikonmaterial bei der Herstellung des Abdrucks und verhindern, damit daß sich im Bereich des Meßfeldes ein die Meßoberfläche verändernder Druck aufbauen kann. Gleichzeitig wird das Silikonmaterial von der Rückseite des Meßfeldes 4 weggeleitet, so daß kein Silikonmaterial auf die Rückseite des Meßfelds 4 gelangen kann.

Die Abflußkanäle 10 dienen außerdem zur Verankerung des Silikonabdrucks auf der Trägerplatte 2, da der Silikonabdruck auf der Trägerplatte 2 verbleibend ausgewertet wird.

## Patentansprüche

1. Vorrichtung zur Messung einer dreidimensionalen Oberflächenstruktur einer Meßoberfläche (9), insbesondere von Hautfalten einer menschlichen Hautoberfläche, mit einer transparenten Trägerplatte (2) zur Aufnahme von zunächst flüssigem Abdruckmaterial für einen Abdruck der Meßoberfläche (9), bei der die Trägerplatte (2) ein Meßfeld (4) aufweist, das in vorbestimmtem Abstand von der Meßoberfläche (9) gehalten ist, und mit einer Auswerteeinheit zur Auswertung der Oberflächenstruktur des Abdrucks,
**dadurch gekennzeichnet,**
daß das in das Meßfeld (4) einzubringende flüssige Abdruckmaterial aus gleichmäßig eingefärbtem Silikonmaterial (3) besteht, und daß die Trägerplatte (2) mindestens einen von dem Meßfeld (4) wegführenden Abflußkanal (10) für überschüssiges Silikonmaterial (3) aufweist.

2. Vorrichtung nach Anspruch 1, dadurch gekennzeichnet, daß die Trägerplatte (2) aus einer dünnen transparenten Scheibe (5) und einer doppelseitig klebenden Folie (7) besteht, die als Abstandhalter dient und in der das Meßfeld (4) und mindestens ein Abflußkanal (10) ausgestanzt sind.

3. Vorrichtung nach Anspruch 1 oder 2, dadurch gekennzeichnet, daß innerhalb des Meßfelds (4) eine zu der Meßoberfläche (9) gerichtete stufenförmige Kalibriereinrichtung (6) mit mindestens zwei definierten Stufenabständen vorgesehen ist.

4. Vorrichtung nach Anspruch 3, dadurch gekennzeichnet, daß das Silikonmaterial (3) mit einer solchen gleichmäßigen Intensität eingefärbt ist, daß für jeden Stufensprung (8) der Kalibriereinrichtung (6) ein mit hoher Auflösung meßbarer Helligkeitsunterschied entsteht.

5. Vorrichtung nach einem der Ansprüche 1 oder 2, dadurch gekennzeichnet, daß die Lichtabsorption des Silikonmaterials (3) durch Einfärbung derart gewählt ist, daß bezogen auf eine maximale Schichtstärke des Silikonmaterials von ca. 1 mm 256 Helligkeitswerte unterscheidbar sind.

6. Vorrichtung nach einem der Ansprüche 1 bis 3, dadurch gekennzeichnet, daß die Trägerplatte (2) in einem Rahmen (1) angeordnet ist, der in Diaprojektoren und Videoauswerteeinrichtungen einsetzbar ist.

7. Vorrichtung nach einem der Ansprüche 1 bis 6, dadurch gekennzeichnet, daß der Silikonabdruck zur Auswertung auf der Trägerplatte (2) verbleibt.

8. Vorrichtung nach einem der Ansprüche 1 bis 7, dadurch gekennzeichnet, daß vom Rand des Meßfeldes (4) Abflußkanäle (10) durch die Trägerplatte (2) hindurchgeführt sind.

9. Verwendung der Vorrichtung gemäß Ansprüche 1 bis 8 zur Messung einer dreidimensionalen Oberflächenstruktur einer Meßoberfläche (9), insbesondere von Hautfalten einer menschlichen Hautoberfläche, durch Herstellen eines Abdrucks der Meßoberfläche (9) mit Hilfe der transparenten Trägerplatte (2), wobei nach dem Aushärten des Abdruckmaterials die Trägerplatte (2) von der Meßoberfläche (9) abgezogen wird und der gehärtete Abdruck in einer optischen Auswerteeinrichtung für die Oberflächenstruktur ausgewertet wird, wobei als flüssiges Abdruckmaterial ein gleichmäßig eingefärbtes Silikonmaterial (3) verwendet wird, das auf die von dem Meßfeld (4) umgrenzte Meßoberfläche (9) aufgegeben wird, wobei das Meßfeld (4) mit mindestens einem Abflußkanal (10) für überschüssiges flüssiges Silikonmaterial (3) verwendet wird, bei dem überschüssiges Silikonmaterial (3) aus dem Meßfeld (4) über den Abflußkanal (10) entweicht.

## Claims

1. A device for measuring a three-dimensional surface structure of a measurement surface (9), in particular skin folds of a human skin surface, comprising a transparent support plate (2) for receiving an initially liquid impression material for an impression of the measurement surface (9), wherein the support plate (2) comprises a measurement zone (4) held at a given distance from the measurement surface (9), and comprising an analyzing unit for analyzing the surface structure of the impression,
**characterized in**
that the liquid impression material to be introduced into the measurement zone (4) comprises a uniformly colored silicone material (3), and that the support plate (2) comprises at least one drainage channel (10) for excess silicone material (3) leading away from the measurement zone (4).

2. The device according to claim 1, characterized in that the support plate (2) consists of a thin transparent pane (5) and a double-sided adhesive sheet (7) which serves as spacer and in which the measurement zone (4) and at least one drainage channel (10) are punched out.

3. The device according to claim 1 or 2, characterized in that a step-shaped calibrating means (6) directed to the measurement surface (9) and comprising at least two defined step distances is provided within the measurement zone (4).

4. The device according to claim 3, characterized in that the silicone material (3) is colored with such a uniform intensity that a brightness difference measurable with high resolution arises for each amount of a step (8) of the calibrating means (6).

5. The device according to one of claims 1 or 2, characterized in that the light absorption of the silicone material (3) is selected, through coloring, in such a manner that 256 brightness values can be distinguished with respect to a maximum layer thickness of the silicone material of about 1 mm.

6. The device according to one of claims 1 to 3, characterized in that the support plate (2) is arranged in a frame (1) which can be inserted in diascopes and video analyzers.

7. The device according to one of claims 1 to 6, characterized in that the silicone impression remains on the support plate (2) for analysis.

8. The device according to one of claims 1 to 7, characterized in that drainage channels (10) are led through the support plate (2) from the edge of the measurement zone (4).

9. Use of the device according to any one of claims 1 to 8 for measuring a three-dimensional surface structure of a measurement surface (9), in particular skin folds of a human skin surface, by producing an impression of the measurement surface (9) by means of the transparent support plate (2), wherein, after the solidifying of the impression material, the support plate (2) is removed from the measurement surface (9) and the solidified impression is analyzed in an optical analyzing unit for the surface structure, wherein the liquid impression material used is a uniformly colored silicone material (3) which is applied onto the measurement surface (9) surrounded by the measurement zone (4), the measurement zone (4) being used with at least one drainage channel (10) for excess liquid silicone material (3), with excess silicone material (3) being discharged from the measurement zone (4) through the drainage channel (10).

## Revendications

1. Dispositif permettant de mesurer une structure superficielle tridimensionnelle d'une surface de mesure (9), en particulier des plis de la peau d'un épiderme humain, comprenant une plaque support (2) transparente pour la réception d'une matière de prise d'empreinte tout d'abord liquide pour une empreinte de la surface de mesure (9), dans lequel la plaque support (2) présente un champ de mesure (4) qui est maintenu à une distance prédéterminée de la surface de mesure (9), et comprenant une unité d'évaluation pour évaluer la structure superficielle de l'empreinte, caractérisé par le fait que la matière liquide de prise d'empreinte à introduire dans le champ de mesure (4) se compose d'une matière silicone (3) uniformément colorée, et que la plaque support (2) comporte au moins un canal d'écoulement (10), partant du champ de mesure (4), pour le matière silicone (3) excédentaire.

2. Dispositif selon la revendication 1, caractérisé par le fait que la plaque support (2) se compose d'une lame mince transparente (5) et d'une feuille (7) collante sur les deux faces, qui sert de support d'écartement et dans laquelle sont découpés le champ de mesure (4) et au moins un canal d'écoulement (10).

3. Dispositif selon la revendication 1 ou 2, caractérisé par le fait qu'un dispositif de calibrage (6) en forme de gradins dirigé vers la surface de mesure (9), avec au moins deux écarts de gradin définis, est prévu à l'intérieur du champ de mesure (4).

4. Dispositif selon la revendication 3, caractérisé par le fait que la matière silicone (3) est colorée avec une intensité si uniforme que pour chaque saut de gradin (8) du dispositif de calibrage (6) se produit une différence de luminosité mesurable avec une haute résolution.

5. Dispositif selon l'une des revendications 1 ou 2, caractérisé par le fait que l'absorption de lumière de la matière silicone (3) par la coloration est choisie de telle sorte que 256 valeurs de luminosité sont différenciables concernant une épaisseur de couche maximale de matière silicone d'environ 1 mm.

6. Dispositif selon l'une des revendications 1 à 3, caractérisé par le fait que la plaque support (2) est disposée dans un cadre (1) qui peut être placé dans des projecteurs de diapositive ou dans des dispositifs d'évaluation vidéo.

7. Dispositif selon l'une des revendications 1 à 6, caractérisé par le fait que l'empreinte de silicone demeure sur la plaque support (2) pour l'évaluation.

8. Dispositif selon l'une des revendications 1 à 7, caractérisé par le fait que des canaux d'écoulement (10) traversent la plaque support (2) à partir du bord du champ de mesure (4).

9. Utilisation du dispositif selon les revendications 1 à 8, pour la mesure d'une structure superficielle tridimensionnelle d'une surface de mesure (9), en particulier des plis de la peau d'un épiderme humain, par préparation d'une empreinte de la surface de mesure (9) à l'aide de la plaque support transparente (2), la plaque support (2) étant retirée de la surface de mesure (9) après durcissement de la matière de prise d'empreinte, et l'empreinte durcie étant évaluée quant à la structure superficielle dans un dispositif d'évaluation optique, utilisation dans laquelle une matière silicone (3) uniformément colorée est utilisée comme matière de prise d'empreinte liquide, qui est mise en place sur la surface de mesure (9) délimitée par le champ de mesure (4), le champ de mesure (4) étant utilisé avec au moins un canal d'écoulement (10) pour la matière silicone (3) liquide excédentaire, cette matière silicone (3) excédentaire s'échappant du champ de mesure (4) par le canal d'écoulement (10).
